# FASCICULE DE BREVET EUROPEEN

(11) **EP 2 501 690 B1**
(45) Date de publication et mention de la délivrance du brevet: **12.04.2017**
(21) Numéro de dépôt: 10803598.1
(22) Date de dépôt: 19.11.2010
(51) Int. Cl.: C07D 317/36, C07D 317/38, C08G 18/32, C08G 59/22, C08G 71/04

(54) **PRÉCURSEURS BISCARBONATES, LEUR PROCÉDÉ DE PRÉPARATION ET LEURS UTILISATIONS**
BICARBONATVORLÄUFER, VERFAHREN ZU IHRER HERSTELLUNG UND VERWENDUNG
BICARBONATE PRECURSORS, METHOD FOR PREPARING SAME AND USES THEREOF

(30) Priorité: 20.11.2009 FR 0958219
(43) Date de publication de la demande: 26.09.2012
(73) Titulaire: Centre National de la Recherche Scientifique (C.N.R.S.), 75016 Paris (FR)
(72) Inventeur: CRAMAIL, Henri, F-33350 Sainte Terre (FR); BOYER, Aurélie, F-33000 Bordeaux (FR); CLOUTET, Eric, F-33880 Saint Caprais De Bordeaux (FR); GADENNE, Benoit, F-33140 Villenave D'ornon (FR); ALFOS, carine, F-33600 Pessac (FR)
(74) Mandataire: Lavoix
(86) Numéro de dépôt international: PCT/FR2010/052457
(87) Numéro de publication internationale: WO 2011/061452

(56) Documents cités:
- EP-A1- 0 001 088
- EP-A1- 0 661 354
- EP-A2- 0 212 409
- EP-A2- 0 229 622
- WO-A1-2007/055929
- US-A- 4 542 173
- US-A- 4 808 658
- WEBSTER ET AL: "CYCLIC CARBONATE FUNCTIONAL POLYMERS. SYNTHESIS AND APPLICATIONS", POLYMER NEWS, GORDON AND BREACH, NEW YORK, NY, US, vol. 23, no. 6, 1 janvier 1998 (1998-01-01), pages 187-192, XP009078653, ISSN: 0032-3918
- BUERGEL, THOMAS ET AL: "Reaction of cyclic carbonates with amines: linear telechelic oligomers", POLYMER BULLETIN, vol. 30, no. 2, 1993, pages 155-162, XP002599513,
- KIHARA, NOBUHIRO ET AL: "Synthesis and properties of poly(hydroxyurethanes)", JOURNAL OF POLYMER SCIENCE, PART A: POLYMER CHEMISTRY, vol. 31, no. 11, 1993, pages 2765-2773, XP002599514,
- ONDRUSCHKA B ET AL: "Incorporation of CO2 into various terminal and internal Epoxides", COLLECTION OF CZECHOSLOVAK CHEMICAL COMMUNICATIONS, INSTITUTE OF ORGANIC CHEMISTRY & BIOCHEMISTRY, PRAGUE; CZ LNKD- DOI:10.1135/CCCC20080088, vol. 73, no. 1, 1 janvier 2008 (2008-01-01), pages 88-96, XP002581705, ISSN: 0010-0765
- BOBYLEVA L I ET AL: "Condensation of olefin oxides with carbon dioxide in the presence of metal chloride-dimethylformamide catalyst", NEFTEHIMIA/NEFTECHIMIJA, AKADEMIA NAUK SSSR, MOSCOW, RU, vol. 36, no. 3, 1 janvier 1996 (1996-01-01), pages 209-213, XP009132962, ISSN: 0028-2421
- GABRIEL ROKICKI ET AL: "Studies on the synthesis of poly(hydroxyurethanes)s from diepoxides carbon dioxide and diamines", POLIMERY, INSTYTUT CHEMII PRZEMYSOWEJ, WARSAW, PL, vol. 34, no. 4, 1 janvier 1989 (1989-01-01), pages 140/141-147, XP009132957, ISSN: 0032-2725

## Description

La présente invention a pour objet des précurseurs biscarbonates ainsi que leur procédé de préparation. Elle a également pour objet leur utilisation pour la préparation de polyuréthanes.

La synthèse de polyuréthanes linéaires se fait par la réaction entre un diol et un diisocyanate. Les isocyanates sont des composés toxiques qui sont obtenus à partir du phosgène, lui-même très toxique par inhalation et provoquant des brûlures. Le procédé utilisé industriellement est la réaction d'une amine avec un excès de phosgène pour former un isocyanate et un mélange de chlorure d'hydrogène et de phosgène.

La recherche d'alternatives à la synthèse de polyuréthanes sans isocyanates représente donc un enjeu majeur.

A ce jour, la carbonatation de triglycérides est connue. Or la carbonatation de triglycérides conduit à l'obtention de synthons possédant un nombre mal défini de groupements carbonates cycliques. La fonctionnalité de ces précurseurs n'est donc pas contrôlée et est supérieure à 2.

La présente invention a pour but de fournir des synthons présentant une fonctionnalité contrôlée.

La présente invention a pour but de fournir des synthons présentant une fonctionnalité exactement égale à 2.

Un autre but de la présente invention est la synthèse de polymères linéaires à structures et propriétés contrôlées à partir de synthons présentant une fonctionnalité contrôlée et exactement égale à 2.

Ainsi, la présente invention concerne des composés de formule (I) suivante : 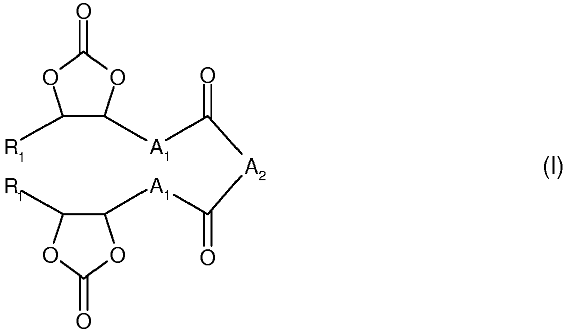 dans laquelle :
- R₁ représente H ou un groupe alkyle, linéaire ou ramifié, comprenant de 1 à 20 atomes de carbone,
- A₁ représente un radical alkylène linéaire comprenant 7 atomes de carbone , et
- A₂ représente un radical -O-A₄-O-, A₄ représentant un radical alkylène divalent linéaire ou ramifié, comprenant de 1 à 20 atomes de carbone, le cas échéant étant interrompu par un ou plusieurs groupes choisis parmi le radical phénylène et le radical de formule -(CH₂OCH₂)ₙ-, n représentant un nombre entier compris de 1 à 100.

WO2007/055929 concerne également des carbonates cycliques polyfonctionnels. Toutefois, les composés de formule générale (I) selon l'invention se différencient des composés décrits dans WO2007/055929 en ce qu'ils comprennent un groupe de structure -A₁-CO-O-A₄-O-CO-A₁- reliant les carbones cycliques, avec A₁ et A₄ étant tels que définis ci-dessus.

Il est également décrit des composés de formule (I) suivante : 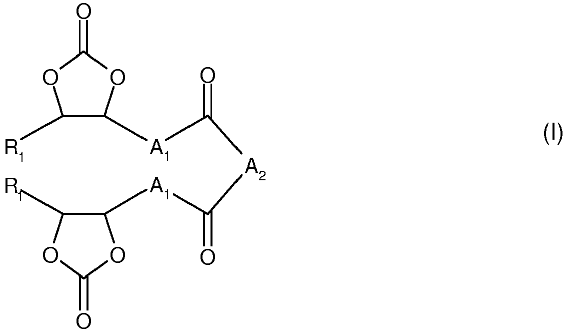 dans laquelle :
- R₁ représente H ou un groupe alkyle, linéaire ou ramifié, comprenant de 1 à 20 atomes de carbone,
- A₁ représente un radical alkylène divalent, linéaire ou ramifié, comprenant de 2 à 14 atomes de carbone, et
- A₂ représente un radical -O-A₄-O-, A₄ représentant un radical alkylène divalent linéaire ou ramifié, comprenant de 1 à 20 atomes de carbone, de préférence de 1 à 10 atomes de carbone, le cas échéant comprenant un ou plusieurs substituants, ou étant interrompu par un ou plusieurs groupes, notamment choisis dans le groupe constitué du radical phénylène et du radical de formule -(CH₂OCH₂)ₙ-, n représentant un nombre entier compris de 1 à 100, de préférence de 6 à 50, et préférentiellement égal à 6, 13 ou 45, ou A₂ représente un radical de formule -(OCH₂CH₂)ₙ-O-, n étant tel que défini ci-dessus.

Il est également décrit que le radical A₄ susmentionné peut représenter un radical de formule -CH₂-A₃-CH₂-, A₃ représentant un groupe de formule - (CH₂OCH₂)ₙ-, n représentant un nombre entier compris de 1 à 100, et de préférence égal à 6, 13 ou 45, ou un radical phénylène.

Selon un mode de réalisation préféré, les composés de la présente invention répondent à la formule (I-1) suivante :

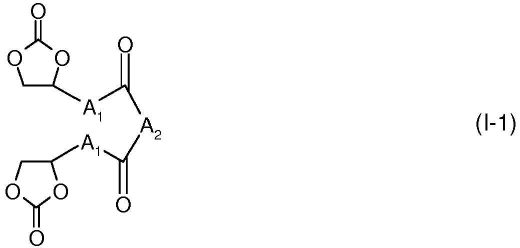

A₁ et A₂ étant tels que définis ci-dessus dans la formule (I).

Ces composés sont donc des composés de formule (I) telle que définie ci-dessus dans laquelle R₁ est H.

Une autre famille de composés préférés selon l'invention est constituée de composés de formule (I) telle que définie ci-dessus, dans laquelle R₁ est un groupe alkyle, linéaire ou ramifié, comprenant de 1 à 20 atomes de carbone, et de préférence de 1 à 12 atomes de carbone.

Selon un autre mode de réalisation particulier, dans la formule (I) susmentionnée, A₄ représente un radical alkylène linéaire comprenant 3, 4, 5 ou 6 atomes de carbone.

Une autre famille de composés préférés selon l'invention est constituée de composés de formule (I-2) telle que définie ci-dessous,

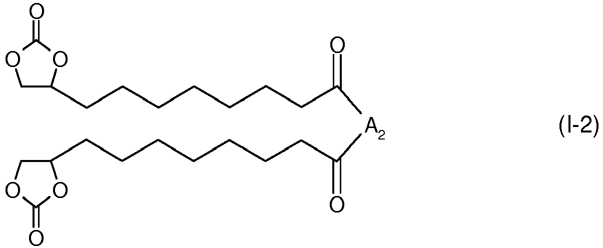

A₂ étant tel que défini ci-dessus.

Une autre famille de composés préférés selon l'invention est constituée de composés de formule (I-3) telle que définie ci-dessous,

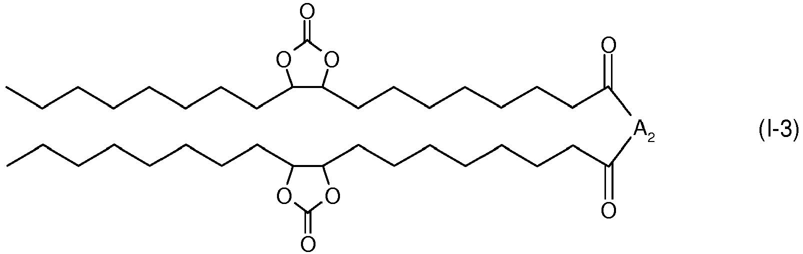

A₂ étant tel que défini ci-dessus.

La présente invention concerne également un procédé de préparation du composé de formule (I) tel que défini ci-dessus, comprenant une étape de carbonatation d'un composé de formule (II) suivante : 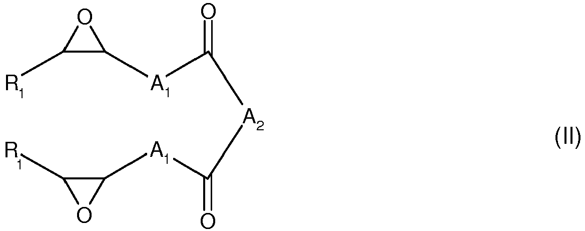 A₁, A₂ et R₁ étant tels que définis dans la formule (I), et R₁ représentant de préférence H.

De préférence, cette étape de carbonatation est effectuée en présence de CO₂ sous toute forme, par exemple sous forme liquide, gazeuse ou supercritique (selon la pression) et d'un réactif choisi parmi le bromure de tétrabutylammonium, l'hydroxyde de tétrabutylammonium et les mélanges de tétrachlorures d'étain (SnCl₄:5H₂O). Elle est effectuée préférentiellement en présence de CO₂ supercritique et de bromure de tétrabutylammonium.

Selon un mode de réalisation avantageux, l'étape de carbonatation susmentionnée est effectuée à une température comprise de 80°C à 150°C, et préférentiellement à 120°C. Lorsque la température est inférieure à 80°C, la cinétique est beaucoup trop lente et l'énergie d'activation est insuffisante, et lorsque la température est supérieure à 150°C, on observe une dégradation du catalyseur.

De façon préférée, lors de l'étape de carbonatation susmentionnée, le dioxyde de carbone est introduit à une pression comprise de 100 bar à 200 bar.

Selon un mode de réalisation particulièrement avantageux, le composé de formule (II) telle que définie ci-dessus est préparé par époxydation du composé de formule (III) suivante : 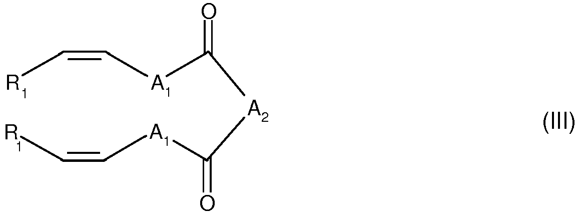 A₁, A₂ et R₁ étant tels que définis dans la formule (I).

Selon un mode de réalisation préféré, l'étape b) du procédé de l'invention est effectuée en présence d'un peracide, cette étape étant notamment effectuée sous vide à 200°C pendant moins de 3 minutes.

Parmi les peracides, on peut notamment citer l'acide métachloroperbenzoïque (m-CPBA) et le peracide magnésium monoperoxyphthalate hexahydrate (MMPP).

La spécificité de ce procédé consiste en l'utilisation de peracide préformé.

Selon un mode de réalisation particulièrement avantageux, le composé de formule (III) telle que définie ci-dessus est préparé par transestérification d'un composé de formule (IV) suivante : 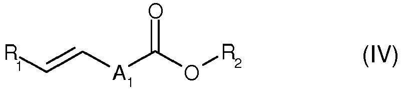 avec un diol de formule (V) suivante :

H-A₂-H (V)

R₁, A₁ et A₂ étant tels que définis dans la formule (I), et
R₂ représentant un groupe alkyle, linéaire ou ramifié, comprenant de 1 à 10, de préférence de 1 à 6, atomes de carbone.

Le procédé de l'invention de préparation des composés (III) consiste en une étape de transestérification effectuée en catalyse hétérogène (oxyde de magnésium ou autre catalyseur hétérogène) et de préférence en l'absence de solvant (synthèse propre).

Selon un mode de réalisation préféré, cette étape de transestérification est effectuée en présence d'un catalyseur choisi dans le groupe constitué de l'oxyde de magnésium, de l'acétate de zinc et du méthanolate de sodium.

De préférence, cette étape est effectuée à une température comprise de 150 à 200°C sous flux d'azote. Cette gamme de température est choisie en fonction de la nature du catalyseur utilisé. Par exemple, si la température est supérieure à 200°C, le catalyseur est dégradé.

Selon un mode de réalisation préféré, cette étape est effectuée sans solvant, ce qui est très avantageux en termes d'écologie.

La présente invention concerne donc un procédé de préparation d'un composé de formule (I) telle que définie ci-dessus, comprenant les étapes suivantes :
a) une étape de transestérification d'un composé de formule (IV) suivante : 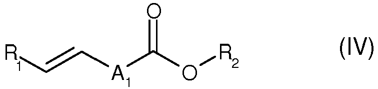 avec un diol de formule (V) suivante :

   H-A₂-H (V)

   R₁, A₁ et A₂ étant tels que définis dans la formule (I), et
   R₂ représentant un groupe alkyle, linéaire ou ramifié, comprenant de 1 à 10, de préférence de 1 à 6, atomes de carbone,
   pour obtenir un composé de formule (III) suivante : 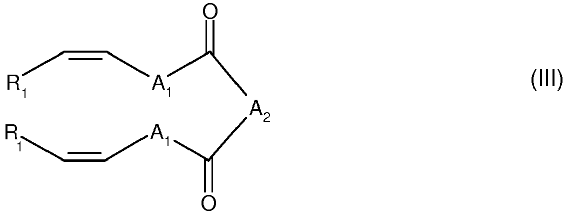 R₁, A₁ et A₂ étant tels que définis dans la formule (I),
b) une étape d'époxydation du composé de formule (III) susmentionnée pour obtenir un composé de formule (II) suivante : 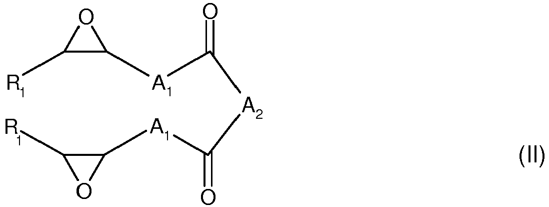 R₁, A₁ et A₂ étant tels que définis dans la formule (I),
c) une étape de carbonatation du composé de formule (II) susmentionnée pour obtenir un composé de formule (I) telle que définie ci-dessus, et
d) une étape de récupération du composé de formule (I) telle que définie ci-dessus.

Le procédé susmentionné selon l'invention peut également comprendre une étape intermédiaire entre l'étape a) et l'étape b) qui consiste à purifier les composés de formule (III), notamment sur une colonne de silice ou par distillation sous vide poussé.

Le procédé susmentionné selon l'invention peut également comprendre une étape intermédiaire entre l'étape b) et l'étape c) qui consiste à purifier les composés de formule (II), notamment sur une colonne de silice.

### Description détaillée des étapes du procédé de l'invention

### 1. Réaction de transestérification des composés de formule (IV)

Dans le cadre du procédé de l'invention, cette transestérification s'effectue de préférence à partir d'un ester d'alcool léger (notamment méthanol ou éthanol...) de tournesol oléique (composé de formule (IV)) et d'un diol (composé de formule (V)) en présence notamment d'oxyde de magnésium comme catalyseur. Plusieurs synthèses sont réalisées avec différents diols afin de moduler les propriétés des monomères et donc des polymères en résultant. Des transestérifications ont donc été réalisées à partir de propanediol, de butanediol, de pentanediol, d'hexanediol, de polyoxyde d'éthylène et de 1,4-benzènediméthanol.

La réaction se déroule entre 150°C et 200°C sous flux d'azote. L'avancement de la réaction est suivi par différentes analyses et notamment la RMN (disparition du singulet du groupement méthyle).

Le diol est introduit en défaut (par exemple 0,5 mol pour 1 mol de composé de formule (IV)).

La voie de synthèse mise en jeu est « propre » car elle fait appel à une catalyse hétérogène (oxyde de magnésium) et la synthèse se déroule sans solvant. La purification industrielle peut se faire par distillation sous vide poussé.

### 2. Epoxydation des composés obtenus après transestérification

L'époxydation de corps gras possédant un alcool primaire en bout de chaîne par des *per*acides formés *in situ* n'a jamais été traitée et ne peut pas être réalisée dans les mêmes conditions que celles décrites précédemment. La réaction d'époxydation est en effet parasitée par une réaction secondaire d'oxydation de l'alcool terminal du monoester de tournesol oléique pour former un acide carboxylique. Le réactif étant consommé par cette réaction secondaire, l'époxydation ne se réalise pas.

La méthode mise en oeuvre dans le cadre du procédé de l'invention pour l'époxydation des composés de formule (III) réside dans l'utilisation d'un peracide déjà formé et commercialisable, à savoir notamment l'acide métachloroperbenzoïque (m-CPBA), et évite donc l'utilisation de métaux potentiellement toxiques.

Dans le cadre de l'invention, l'époxydation a été réalisée avec des peracides (mCPBA, MMPP...). La réaction peut être suivie par RMN; la disparition des doublets des protons de la double liaison à 5,2 ppm ainsi que l'apparition d'un pic large à 2,8 ppm permettent de suivre l'avancement de la réaction. La conversion des doubles liaisons est totale au bout de 3h. L'excès de m-CPBA est réduit en acide carboxylique correspondant avec une solution saturée de sulfate de sodium. La phase organique est extraite avec du dichlorométhane puis l'acide carboxylique résiduel est transformé en chlorobenzoate de sodium (soluble dans l'eau) grâce à deux lavages avec une solution saturée de bicarbonate de soude.

La présente invention concerne également l'utilisation du composé de formule (I) telle que définie ci-dessus, pour la préparation de polyuréthane.

La synthèse de polyuréthanes à partir d'huiles végétales époxydées et de dioxyde de carbone permet de s'affranchir de l'utilisation d'isocyanates, qui sont des composés toxiques. La réaction en jeu est l'obtention d'un groupement carbonate cyclique grâce à la réaction du groupe époxyde de l'huile végétale avec le CO₂. Lors de la polymérisation, le carbonate réagit alors avec un donneur d'hydrogène, comme par exemple une amine, pour former une fonction uréthane.

La présente invention concerne également les polyuréthanes tels qu'obtenus, par réaction des composés de formule (I) avec un donneur d'hydrogène.

La présente invention concerne également l'utilisation du composé de formule (II) telle que définie ci-dessus dans laquelle R₁ est H, pour la préparation de résines polyépoxydes.

La présente invention concerne également les résines polyépoxydes tels qu'obtenues, par réaction des composés de formule (II) telle que définie ci-dessus, dans laquelle R₁ est H, avec un donneur d'hydrogène.

### PARTIE EXPÉRIMENTALE

### Exemple 1 : Préparation de biscarbonates à groupements carbonates cycliques internes

Ces composés sont préparés selon le schéma réactionnel suivant :

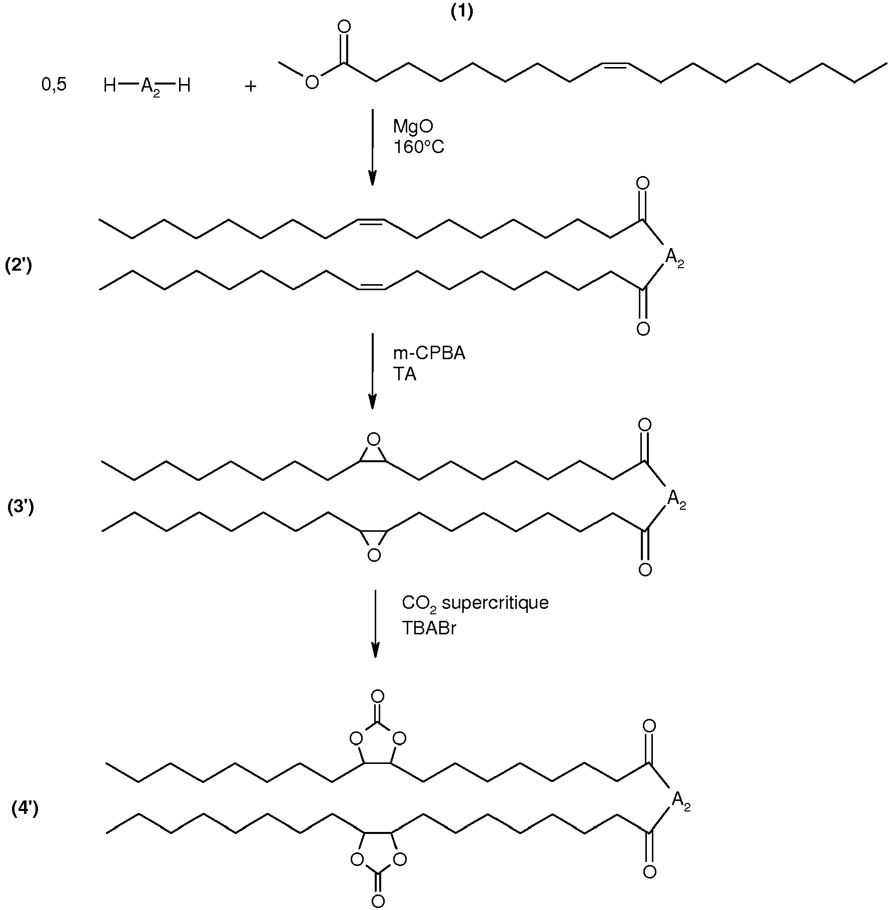

Le composé 4' correspond à un composé de formule (I-3) telle que définie ci-dessus. Dans ce composé, A₂ peut représenter un radical choisi parmi les radicaux suivants : -OC₃H₆O-, -OC₄H₈O-, -OC₅H₁₀O-, -OC₆H₁₂O-, -OH₂C-(CH₂OCH₂)₆-CH₂O-, -OH₂C-(CH₂OCH₂)₁₃-CH₂O-, -OH₂C-(CH₂OCH₂)₄₅-CH₂O- ou -OH₂C-C₆H₄-CH₂O-.

### Etape 1 : synthèse du composé 2'

On procède à une réaction de transestérification de l'ester méthylique oléique (**1**) avec un diol H-A₂-H (A₂ étant tel que défini ci-dessus).

On introduit l'ester méthylique oléique (**1**) dans un ballon de 250mL équipé d'une trappe Dean Stark et surmonté d'un réfrigérant. La réaction se déroule sous vide en présence de 0,5 équivalent de diol afin de favoriser la formation de diesters (**2'**) ; elle est catalysée par 1_{wt}% d'oxyde de magnésium. La température du milieu est élevée à 160°C, le méthanol produit est enlevé en continu à l'aide de la trappe Dean Stark. Au bout de 7h, la température est élevée à 180°C pendant 30 minutes afin d'éliminer d'ester méthylique oléique (**1**) résiduel. Rendement : 95%.

Les protocoles décrits ci-dessus sont les mêmes indépendamment de la nature des diols utilisés pour la transestérification (c'est-à-dire indépendamment de la nature de A₂). Les différents diols utilisés sont : propanediol, butanediol, pentanediol, hexanediol, polyoxyde d'éthylène (300g/mol, 600g/mol et 2 000g/mol) et 1,4-benzènediméthanol.

A noter que la transestérification avec le 1,4-benzènediméthanol est réalisée à 140°C afin d'éviter la sublimation du diol.

### Etape 2 : synthèse du composé 3'

On introduit l'ester méthylique oléique transestérifié (**2'**) dans 100mL de dichlorométhane, dans un ballon de 250mL. On ajoute de l'acide métachloroperbenzoïque (m-CPBA) (1,2eq par double liaison). Le mélange est placé sous agitation à température ambiante. La conversion des doubles liaisons est totale au bout de 3h. L'excès de m-CPBA est réduit en acide carboxylique correspondant avec une solution saturée de sulfate de sodium (100mL). La phase organique est extraite avec du dichlorométhane puis l'acide carboxylique résiduel est transformé en chlorobenzoate de sodium (soluble dans l'eau) grâce à deux lavages avec une solution saturée de bicarbonate de soude (2x 100mL). Rendement : 80%.

### Etape 3 : synthèse du composé 4'

La carbonatation se déroule en présence du composé (**3'**) et de 1_{wt}% de bromure de tétrabutylammonium (TBABr), dans un réacteur haute pression. Le réacteur est chauffé à une température de 120°C puis du dioxyde de carbone est introduit jusqu'à atteindre une pression de 100 bar. La réaction est arrêtée lorsque la conversion des époxydes est totale. Rendement : 90%.

### Exemple 2 : Préparation de biscarbonates à groupements carbonates cycliques terminaux

Ces composés sont préparés selon le schéma réactionnel suivant :

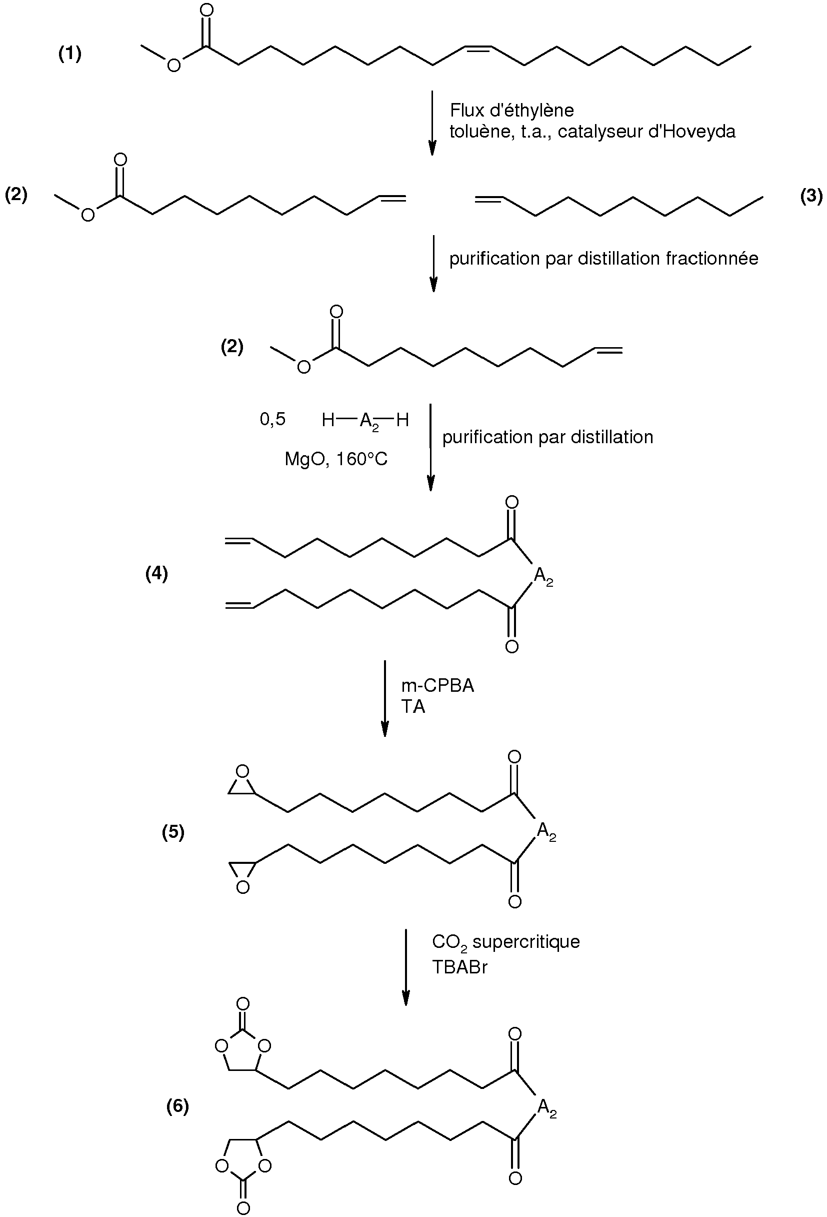

Le composé 6 correspond à un composé de formule (I-2) telle que définie ci-dessus. Dans ce composé, A₂ peut représenter un radical choisi parmi les radicaux suivants : -OC₃H₆O-, -OC₄H₈O-, -OC₅H₁₀O-, -OC₆H₁₂O-, -OH₂C-(CH₂OCH₂)₆-CH₂O-, -OH₂C-(CH₂OCH₂)₁₃-CH₂O-, -OH₂C-(CH₂OCH₂)₄₅-CH₂O- ou -OH₂C-C₆H₄-CH₂O-.

### Etape 1 : synthèse des composés 2 et 3

10g d'ester méthylique oléique sec (3,4.10⁻² mol) (1) sont dissous dans 20mL de toluène distillé, dans un ballon de 250mL. On ajoute 250mg (2,5 _{wt}%) d'un catalyseur Hoveyda et on introduit 1 bar d'éthylène. Le milieu est mis sous agitation magnétique à température ambiante. La réaction est arrêtée au bout de 6h.

### Etape 2 : extraction du composé 2

A l'état d'équilibre, le milieu est composé de 48% d'ester méthylique oléique de départ (**1**), 26% de décène (**3**) et 26% de 10-undécénoate de méthyle (**2**). Ce dernier est extrait par distillation sous vide : la première fraction à 100°C contient du décène (**3**) ; le 10-undécénoate de méthyle (**2**) est récupéré lorsque la température atteint 180°C. Le résidu est composé d'ester méthylique oléique (**1**). Rendement total des étapes 1+2 : 25%.

### Etape 3 : synthèse du composé 4

On procède à une réaction de transestérification du 10-undécénoate de méthyle (**2**) avec un H-A₂-H (A₂ étant tel que défini ci-dessus).

On introduit 2g de 10-undécénoate de méthyle (**1**) dans un ballon de 50mL équipé d'une trappe Dean Stark et surmonté d'un réfrigérant. La réaction se déroule sous vide en présence de 0,5 équivalent de diol afin de favoriser la formation de diesters ; elle est catalysée par 1_{wt}% d'oxyde de magnésium. La température du milieu est élevée à 160°C, le méthanol produit est enlevé en continu à l'aide de la trappe Dean Stark. Au bout de 7h, la température est élevée à 180°C pendant 30 minutes afin d'éliminer le 10-undécénoate de méthyle résiduel. Rendement de l'étape 3 : 90%

Les protocoles décrits ci-dessus sont les mêmes indépendamment de la nature des diols utilisés pour la transestérification (c'est-à-dire indépendamment de la nature de A₂). Les différents diols utilisés sont : propanediol, butanediol, pentanediol, hexanediol, polyoxyde d'éthylène (300g/mol, 600g/mol et 2 000g/mol) et 1,4-benzènediméthanol.

A noter que la transestérification avec le 1,4-benzènediméthanol est réalisée à 140°C afin d'éviter la sublimation du diol.

### Etape 4 : synthèse du composé 5

La dernière étape consiste en l'époxydation des doubles liaisons du produit obtenu par transestérification. On dissout le 10-undécénoate de méthyle transestérifié (**4**) dans 20mL de dichlorométhane, dans un ballon de 50mL. On ajoute de l'acide métachloroperbenzoïque (m-CPBA) (1,2eq par double liaison). Le mélange est placé sous agitation à température ambiante. La conversion des doubles liaisons est totale au bout de 3h. L'excès de m-CPBA est réduit en acide carboxylique correspondant avec une solution saturée de sulfate de sodium (30mL). La phase organique est extraite avec du dichlorométhane puis l'acide carboxylique résiduel est transformé en chlorobenzoate de sodium (soluble dans l'eau) grâce à deux lavages avec une solution saturée de bicarbonate de soude (2x 40mL). Rendement de l'étape 4 : 85%

La polycondensation entre le synthon bis-époxyde et diverses diamines conduit aux matériaux polyépoxydes.

### Etape 5 : synthèse du composé 6

La carbonatation se déroule en présence du composé (**5**) et de 1_{wt}% de bromure de tétrabutylammonium (TBABr), dans un réacteur haute pression. La température du réacteur est élevée à 120°C puis du dioxyde de carbone est introduit jusqu'à atteindre une pression de 100 bar. La réaction est arrêtée lorsque la conversion des époxydes est totale. Rendement : 90%

### Exemple 3 : Synthèse de polymères à partir des différents précurseurs biscarbonates (terminaux et internes)

1 g de précurseur biscarbonates (**4'**) ou (**6**) est ajouté à une diamine (ex : éthylènediamine (EDA), isophorone diamine (IPDA), hexaméthylènediamine (HMDA), aminoéthyl-pipérazine (AEP), diéthylènetriamine), dans un ballon de 50mL. Le nombre de groupements amines est introduit en proportion stoechiométrique par rapport au nombre de fonctions carbonates du composé (4') ou (6). Le mélange est placé sous agitation magnétique, à 70°C. La disparition du groupement carbonate est suivie par analyse infrarouge (disparition de la bande à 1 803 cm⁻¹). La réaction est arrêtée au bout de 8h. La masse molaire du polymère est obtenue par chromatographie d'exclusion stérique et sa température de transition vitreuse est mesurée par calorimétrie différentielle (Differential Scanning Calorimetry).

### Exemple avec A₂ = -OC₅H₁₀O- :

1g de (**4'**) est introduit en présence de 0,079 g de EDA dans un ballon de 50mL. Le mélange est placé sous agitation magnétique, à 70°C. La réaction est arrêtée au bout de 8h. La masse molaire du polymère est obtenue par chromatographie d'exclusion stérique et sa température de transition vitreuse est mesurée par Differential Scanning Calorimetry.

Le même protocole est appliqué pour A₄ = PEG300 et -CH₂-C₆H₄-CH₂- (cf. formule (I)).

Le tableau ci-après donne les résultats (température de transition vitreuse et masse molaire) :

| Polymères à partir de biscarbonates (4') et EDA | T_{g} (°C) | M_{w} (g/mol) |
|---|---|---|
| A₄ = PEG₃₀₀ | -55 | 12 200 |
| A₄ = C₅H₁₀ | -20 | 14 500 |
| 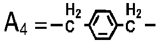 | -15 | 13 400 |

### Exemple 4 : Synthèse de résines polyépoxydes à partir des précurseurs bisépoxydes (composés de formule (II) avec R₁ = H).

La polycondensation entre le synthon bis-époxyde et diverses diamines conduit aux matériaux polyépoxydes.

### Exemple de résines polyépoxydes :

0,5g de composé (5) (1,06e⁻³ mol) sont introduits dans une coupelle en aluminium avec de la diéthylènetriamine (ou éthylènediamine (EDA), hexaméthyldiamine (HMDA), isophoronediamine (IPDA) et aminoéthyl pipérazine AEP). Le nombre de groupements amines est introduit en proportion stoechiométrique par rapport au nombre de fonctions époxydes du composé (5). Le mélange est ensuite introduit dans un moule rectangulaire (longueur 1 cm, épaisseur et largeur 0,5 cm) puis placé dans un four à 90°C pendant 24h. La température de transition vitreuse des résines synthétisées est déterminée par Differential Scanning Calorimetry.

Le tableau ci-après donne les résultats obtenus (températures de transition vitreuse des résines époxy issues de la polycondensation entre le composé (5) et différentes di- ou triamines) :

| Résines époxy à partir de bisépoxydes | EDA | HMDA | DETA | IPDA | AEP |
|---|---|---|---|---|---|
| A₄ = PEG₃₀₀ | -60°C | -52°C | -50°C | -47°C | -46°C |
| A₄ = C₅H₁₀ | -17°C | -17°C | | | |
| A₄ = - CH₂-C₆H₄-CH₂- | -9°C | 2°C | 0°C | 5°C | |

### Exemple de résines époxy formées à partir d'un mélange de diglycidyl éther de bisphénol A (DGEBA) et du composé (5).

avec A₂= -OC₅H₁₀O-

0,48g de DGEBA (M_{DGEBA}= 340,4g/mol) sont mélangés dans une coupelle en aluminium avec 0,12g de composé 5 (R= C₅H₁₀). On rajoute 0,068g de DETA. Le mélange est ensuite introduit dans un moule rectangulaire (longueur 1cm, épaisseur et largeur 0,5cm) puis placé dans un four à 90°C pendant 24h.

Le même protocole est appliqué en changeant les proportions en DGEBA.

Les températures de transition vitreuse des résines synthétisées sont déterminées par Differential Scanning Calorimetry.

Les résultats obtenus sont indiqués ci-après :

| Proportion en DGEBA | T_{g} de la résine époxy (°C) |
|---|---|
| 100% | 120 |
| 80% | 95 |
| 60% | 70 |
| 40% | 55 |
| 20% | 15 |
| 0% | 0 |

## Revendications

1. Composé de formule (I) suivante : 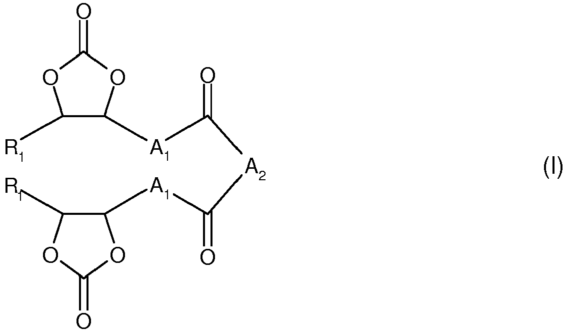 dans laquelle :
- R₁ représente H ou un groupe alkyle, linéaire ou ramifié, comprenant de 1 à 20 atomes de carbone,
- A₁ représente un radical alkylène linéaire comprenant 7 atomes de carbone , et
- A₂ représente un radical -O-A₄-O-, A₄ représentant un radical alkylène divalent linéaire ou ramifié, comprenant de 1 à 20 atomes de carbone, le cas échéant étant interrompu par un ou plusieurs groupes choisis parmi le radical phénylène et le radical de formule -(CH₂OCH₂)ₙ-, n représentant un nombre entier compris de 1 à 100,

2. Composé selon la revendication 1, dans lequel A₂ représente un radical de formule -(OCH₂CH₂)ₙ-O-, n étant tel que défini dans la revendication 1.

3. Composé selon la revendication 1 ou 2, dans lequel R₁ est H.

4. Composé selon l'une quelconque des revendications 1 à 3, dans lequel R₁ est un groupe alkyle, linéaire ou ramifié, comprenant de 1 à 20 atomes de carbone.

5. Procédé de préparation du composé de formule (I) selon l'une quelconque des revendications 1 à 4, comprenant une étape de carbonatation d'un composé de formule (II) suivante : 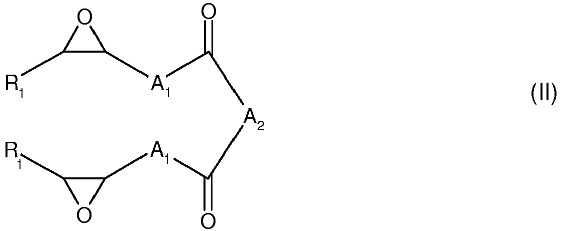 A₁, A₂ et R₁ étant tels que définis dans la revendication 1.

6. Procédé de préparation du composé de formule (I) selon la revendication 5, **caractérisé en ce que** l'étape de carbonatation est effectuée en présence de CO₂ supercritique, et de bromure de tétrabutylammonium.

7. Procédé de préparation du composé de formule (I) selon la revendication 6, **caractérisé en ce que** l'étape de carbonatation est effectuée à une température comprise de 80°C à 150°C.

8. Procédé de préparation du composé de formule (I) selon l'une quelconque des revendications 5 ou 6, **caractérisé en ce que** le composé de formule (II) est préparé par époxydation du composé de formule (III) suivante : 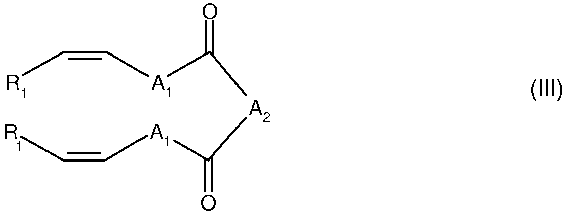 A₁, A₂ et R₁ étant tels que définis dans la revendication 1.

9. Procédé selon la revendication 8, dans lequel l'étape d'époxydation est effectuée en présence d'un peracide.

10. Procédé selon la revendication 8, dans lequel le peracide est choisi dans le groupe constitué de l'acide métachloroperbenzoïque (m-CPBA) et du peracide magnésium monoperoxyphthalate hexahydrate (MMPP).

11. Procédé selon la revendication 9, dans lequel le composé de formule (III) est préparé par transestérification d'un composé de formule (IV) suivante : 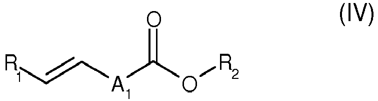 avec un diol de formule (V) suivante :
H-A₂-H (V)
R₁, A₁ et A₂ étant tels que définis dans la revendication 1, et
R₂ représentant un groupe alkyle, linéaire ou ramifié, comprenant de 1 à 10 atomes de carbone.

12. Procédé selon la revendication 11, dans lequel l'étape de transestérification est effectuée en présence d'un catalyseur choisi dans le groupe constitué de l'oxyde de magnésium, de l'acétate de zinc et du méthanolate de sodium.

13. Procédé selon l'une quelconque des revendications 11 à 12, dans lequel l'étape de transestérification est effectuée à une température comprise de 150 à 200°C sous flux d'azote.

14. Procédé selon l'une quelconque des revendications 11 à 13, dans lequel l'étape de transestérification est effectuée sans solvant.

15. Utilisation du composé de formule (I) selon l'une quelconque des revendications 1 à 4, pour la préparation de polyuréthane.

## Patentansprüche

1. Verbindung folgender Formel: 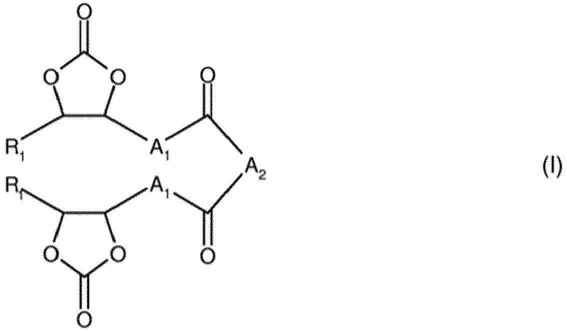 wobei:
- R₁ H oder eine Alkylgruppe, linear oder verzweigt, darstellt, umfassend 1 bis 20 Kohlenstoffatome,
- A₁ ein lineares Alkylenradikal darstellt, umfassend 7 Kohlenstoffatome, und
- A₂ ein Radikal -O-A₄-O- darstellt, wobei A₄ ein zweiwertiges Alkylenradikal, linear oder verzweigt, darstellt, umfassend 1 bis 20 Kohlenstoffatome, gegebenenfalls unterbrochen von einer oder mehreren Gruppen, ausgewählt aus dem Phenylenradikal und dem Radikal der Formel -(CH₂OCH₂)ₙ-, wobei n eine Ganzzahl zwischen 1 bis 100 inklusive darstellt.

2. Verbindung nach Anspruch 1, wobei A2 ein Radikal der Formel -(OCH₂CH₂)ₙ-O-darstellt, wobei n nach Anspruch 1 ist.

3. Verbindung nach Anspruch 1 oder 2, wobei R₁ H ist.

4. Verbindung nach einem der Ansprüche 1 bis 3, wobei R₁ eine Alkylgruppe, linear oder verzweigt, ist, umfassend 1 bis 20 Kohlenstoffatome.

5. Herstellungsverfahren der Verbindung von Formel (I) nach einem der Ansprüche 1 bis 4, umfassend einen Karbonatationsschritt einer Verbindung der folgenden Formel (II): 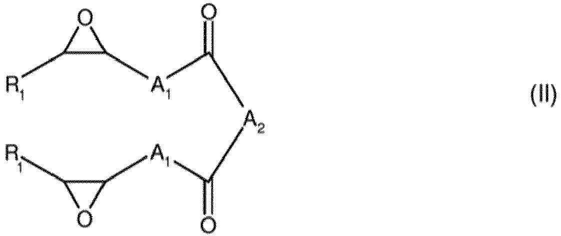 wobei A₁, A₂ und R₁ nach Anspruch 1 sind.

6. Herstellungsverfahren der Verbindung der Formel (I) nach Anspruch 5, **dadurch gekennzeichnet, dass** der Karbonatationsschritt bei Anwesenheit von superkritischem CO₂ und von Tetrabutylammoniumbromid durchgeführt wird.

7. Herstellungsverfahren der Verbindung der Formel (I) nach Anspruch 6, **dadurch gekennzeichnet, dass** der Karbonatationsschritt bei einer Temperatur von 80 °C bis 150 °C durchgeführt wird.

8. Herstellungsverfahren der Verbindung der Formel (I) nach einem der Ansprüche 5 oder 6, **dadurch gekennzeichnet, dass** die Verbindung der Formel (II) durch Epoxidierung der Verbindung der folgenden Formel (III) durchgeführt wird: 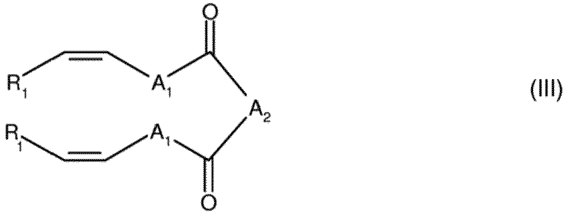 wobei A₁, A₂ und R₁ nach Anspruch 1 sind.

9. Verfahren nach Anspruch 8, wobei der Schritt der Epoxidierung bei Anwesenheit einer Persäure durchgeführt wird.

10. Verfahren nach Anspruch 8, wobei die Persäure aus der Gruppe ausgewählt ist, die von der Metachlorperbenzolsäure (m-CPBA) und dem Magnesium monoperoxyphthalat Hexahydrat (MMPP) gebildet ist.

11. Verfahren nach Anspruch 9, wobei die Verbindung der Formel (III) durch Transesterifizierung einer Verbindung der folgenden Formel (IV) hergestellt wird: 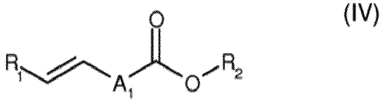 mit einem Diol der folgenden Formel (V):
H-A₂-H (V)
wobei R₁, A₁ und A₂ nach Anspruch 1 sind und
R₂ eine Alkylgruppe, linear oder verzweigt, darstellt, umfassend 1 bis 10 Kohlenstoffatome.

12. Verfahren nach Anspruch 11, wobei der Schritt der Transesterifizierung bei Anwesenheit eines Katalysators durchgeführt wird, der aus der Gruppe ausgewählt ist, die von dem Magnesiumoxid, dem Zinkacetat und dem Natriummethanolat gebildet ist.

13. Verfahren nach einem der Ansprüche 11 bis 12, wobei der Transesterifizierungsschritt bei einer Temperatur von 150 bis 200 °C unter Stickstofffluss durchgeführt wird.

14. Verfahren nach einem der Ansprüche 11 bis 13, wobei der Schritt der Transesterifizierung ohne Lösungsmittel durchgeführt wird.

15. Verwendung der Verbindung der Formel (I) nach einem der Ansprüche 1 bis 4 zur Herstellung von Polyurethan.

## Claims

1. A compound of following formula (I): 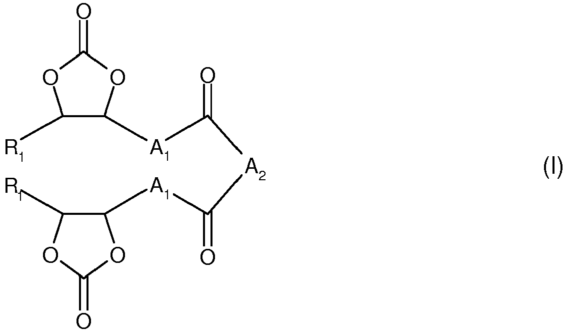 where:
- R₁ is H or a straight-chain or branched alkyl group comprising 1 to 20 carbon atoms,
- A₁ is a divalent straight-chain alkylene radical comprising 7 carbon atoms, and
- A₂ is a -O-A₄-O- radical, A₄ being a divalent straight-chain or branched alkylene radical comprising 1 to 20 carbon atoms, being possibly interrupted by one or more groups chosen from the group formed by the phenylene radical and the radical of formula - (CH₂OCH₂)ₙ-, n representing an integer of between 1 and 100.

2. The compound according to claim 1, wherein A₂ is a radical of formula-(OCH₂CH₂)ₙ-O-, n being such as defined in claim 1.

3. The compound according to claim 1 or 2, where R₁ is H.

4. The compound according to any one of claims 1 to 3, where R₁ is a straight-chain or branched alkyl group comprising 1 to 20 carbon atoms.

5. A method for preparing a compound of formula (I) according to any of claims 1 to 4 comprising a carbonatation step of a compound of following formula (II): 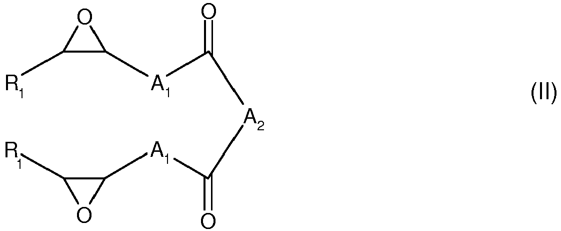 A₁, A₂ and R₁ being such as defined in claim 1.

6. The method for preparing the compound of formula (I) according to claim 5, **characterized in that** the carbonatation step is conducted in the presence of supercritical CO₂ and tetrabutylammonium bromide.

7. The method for preparing the compound of formula (I) according to claim 6, **characterized in that** the carbonation step is conducted at a temperature of between 80°C up to 150°C.

8. The method for preparing the compound of formula (I) according to any one of claims 5 or 6 **characterized in that** the compound of formula (II) is prepared by epoxidation of the compound of following formula (III): 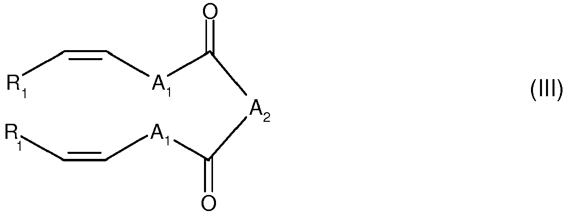 A₁, A₂ and R₁ being such as defined in claim 1.

9. The method according to claim 8, in which the epoxidation step is conducted in the presence of a peracid.

10. The method according to claim 8, wherein the peracid is chosen from the group formed by metachloroperbenzoic acid (m-CPBA) and the peracid magnesium monoperoxyphthalate hexahydrate (MMPP).

11. The method according to claim 9, wherein the compound of formula (III) is prepared by transesterification of a compound of following formula (IV): 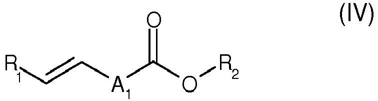 with a diol of following formula (V):
H-A₂-H (V)
R₁, A₁ and A₂ being such as defined in claim 1, and
R₂ representing a straight-chain or branched alkyl group comprising 1 to 10 carbon atoms.

12. The method according to claim 11, in which the transesterification step is conducted in the presence of a catalyst chosen from the group formed by magnesium oxide, zinc acetate and sodium methanolate.

13. The method according to any one of claim 11 or 12, in which the transesterification step is conducted at a temperature of between 150 and 200°C under a flow of nitrogen,

14. The method according to any one of claims 11 to 13, in which the transesterification step is conducted without a solvent.

15. The use of a compound of formula (I) according to any of claims 1 to 4, for the preparation of polyurethane.
